# EUROPEAN PATENT APPLICATION

(11) **EP 4 342 888 A1**
(43) Date of publication of application: **27.03.2024**
(21) Application number: 22804441.8
(22) Date of filing: 31.03.2022
(51) Int. Cl.: C07D 331/02

(54) **THIOEPOXY GROUP- AND (METH)ALLYL GROUP-CONTAINING COMPOUND, AND METHOD FOR PRODUCING SAME**

(30) Priority: 21.05.2021 JP 2021085967
(71) Applicant: SHIN-ETSU CHEMICAL CO., LTD., Tokyo 1000005 (JP)
(72) Inventor: YANAGISAWA Hideyoshi, Annaka-shi, Gunma 379-0224 (JP)
(74) Representative: Ter Meer Steinmeister & Partner
(86) International application number: PCT/JP2022/016405
(87) International publication number: WO 2022/244525

(57) **Abstract**

Provided is a thioepoxy group- and (meth)allyl group-containing compound represented by formula (1). 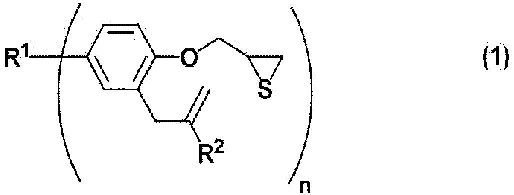 (In the formula, R¹ is a C2-20 trivalent or tetravalent hydrocarbon group. R² is a hydrogen atom or a methyl group, n is 3 or 4.)

## Description

### TECHNICAL FIELD

The present invention relates to a thioepoxy group and (meth)allyl group-containing compound and to a method for preparing the same.

### BACKGROUND ART

The compound described in Patent Document 1 is already known as a (meth)allyl group and glycidyl group-containing compound. Although this compound has an excellent heat resistance and other properties, the refractive index is not high, and so it has been unsatisfactory as a precursor for highly refractive optical materials.

### PRIOR ART DOCUMENTS

### PATENT DOCUMENTS

Patent Document 1: JP-A 2017-125008

### SUMMARY OF INVENTION

### TECHNICAL PROBLEM

The present invention was conceived in light of the above circumstances. The object of the invention is to provide a material which is useful as a macromonomer for heat-resistant resin materials, and moreover which is useful also as a highly refractive optical material that enables the resulting polymer to be made highly refractive.

### SOLUTION TO PROBLEM

The inventor has conducted intensive and repeated investigations aimed at achieving this object, discovering as a result that by reacting a glycidyl group and (meth)allyl group-containing compound of formula (2) below 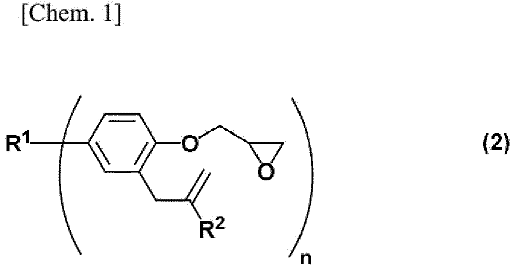 (wherein R¹ is a trivalent or tetravalent hydrocarbon group of 2 to 20 carbon atoms, R² is a hydrogen atom or a methyl group, and n is 3 or 4) with a thiourea or a thiocyanate, a compound of formula (1) below 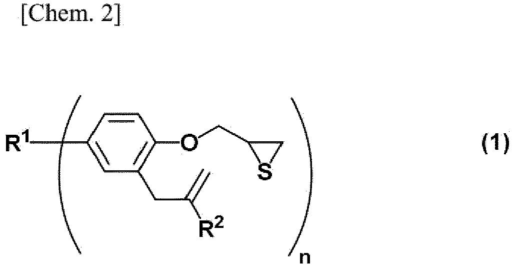 (wherein R¹ is a trivalent or tetravalent hydrocarbon group of 2 to 20 carbon atoms, R² is a hydrogen atom or a methyl group, and n is 3 or 4) having three or more thioepoxy groups and three or more (meth)allyl groups can be obtained. This discovery ultimately led to the present invention.

Accordingly, this invention provides the following thioepoxy group and (meth)allyl group-containing compound, and also provides a method for preparing the same.
1. A thioepoxy group and (meth)allyl group-containing compound of formula (1) below 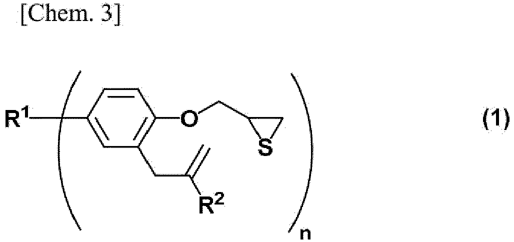 (wherein R¹ is a trivalent or tetravalent hydrocarbon group of 2 to 20 carbon atoms, R² is a hydrogen atom or a methyl group, and n is 3 or 4).
2. The thioepoxy group and (meth)allyl group-containing compound of 1 above, wherein R¹ is a trivalent or tetravalent hydrocarbon group of 2 to 15 carbon atoms.
3. A method for preparing a thioepoxy group and (meth)allyl group-containing compound of formula (1) below 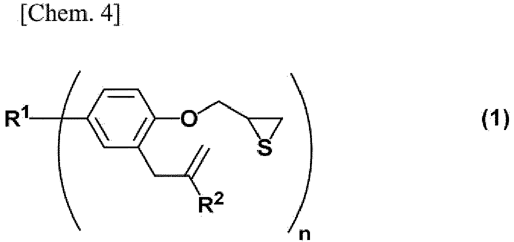 (wherein R¹ is a trivalent or tetravalent hydrocarbon group of 2 to 20 carbon atoms, R² is a hydrogen atom or a methyl group, and n is 3 or 4), which method includes the step of reacting a glycidyl group and (meth)allyl group-containing compound of formula (2) below 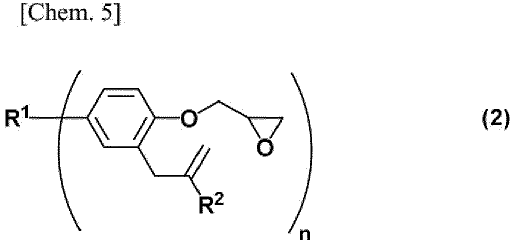 (wherein R¹ is a trivalent or tetravalent hydrocarbon group of 2 to 20 carbon atoms, R² is a hydrogen atom or a methyl group, and n is 3 or 4) with a thiourea or a thiocyanate.

### ADVANTAGEOUS EFFECTS OF INVENTION

Both polymerization using (meth)allyl groups and polymerization using thioepoxy rings are possible with the thioepoxy group and (meth)allyl group-containing compound of the invention and, because the compound has three or more of each of these functional groups per molecule, the resulting polymer can have a three-dimensional crosslinked structure. Also, crosslinking and curing following polymerization using these groups is possible, and so the resulting polymer has a good strength, heat resistance, cold resistance and water resistance. The thioepoxy group and (meth)allyl group-containing compound of the invention is a compound that is useful as a macromonomer for heat-resistant resin materials. In addition, the thioepoxy group and (meth)allyl group-containing compound of the invention, because it has thioepoxy groups, enables the resulting polymer to be made highly refractive, and thus is promising as a useful precursor for highly refractive optical materials.

### DESCRIPTION OF EMBODIMENTS

The thioepoxy group and (meth)allyl group-containing compound of the invention is a compound represented by formula (1) below.

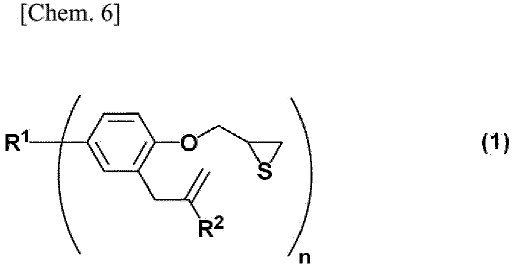

In formula (1), R¹ is a trivalent or tetravalent hydrocarbon group of 2 to 20 carbon atoms. R² is a hydrogen atom or a methyl group, and n is 3 or 4.

The trivalent or tetravalent hydrocarbon group of 2 to 20 carbon atoms may be saturated or unsaturated, and may be linear, branched or cyclic. Specific examples include groups obtained by removing three or four hydrogen atoms from alkanes such as ethane, propane, butane, pentane, hexane and their structural isomers; cyclic saturated hydrocarbons such as cyclopentane, cyclohexane, bicyclohexyl, dicyclohexylmethane, 1,1dicyclohexylethane, 1,2-dicyclohexylethane, 1,1-dicyclohexylpropane, 1,2-dicyclohexylpropane, 1,3-dicyclohexylpropane and 2,2-dicyclohexylpropane; and aromatic hydrocarbons such as benzene, toluene, xylene and ethylisopropylbenzene.

R¹ is preferably a trivalent or tetravalent hydrocarbon group of 2 to 15 carbon atoms. The groups shown below are especially preferred. 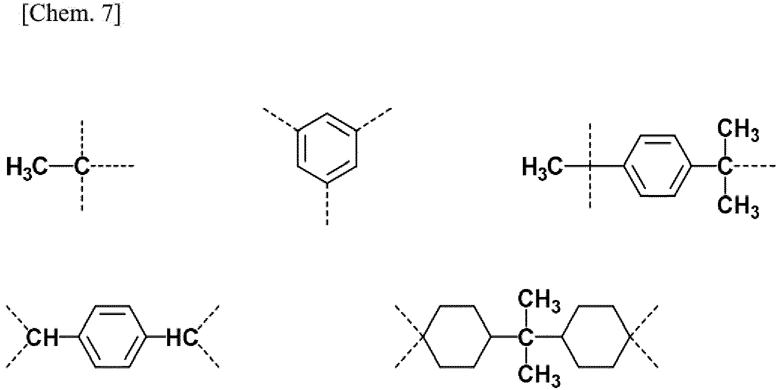 (wherein the dashed lines are available bonds.)

Examples of the compound of formula (1) include, but are not limited to, those shown below.

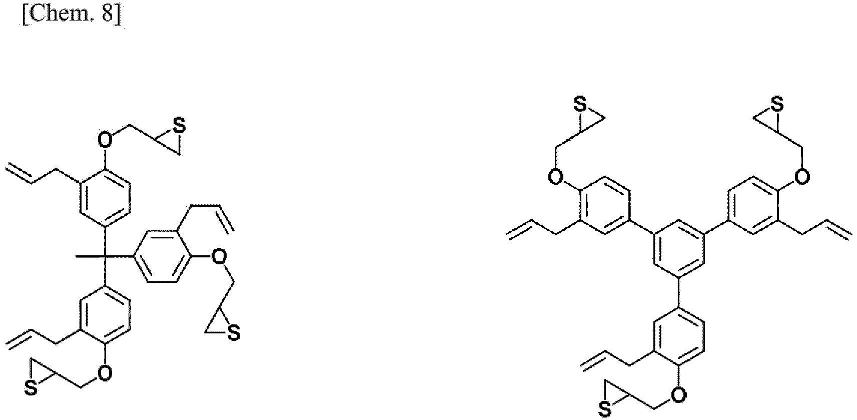

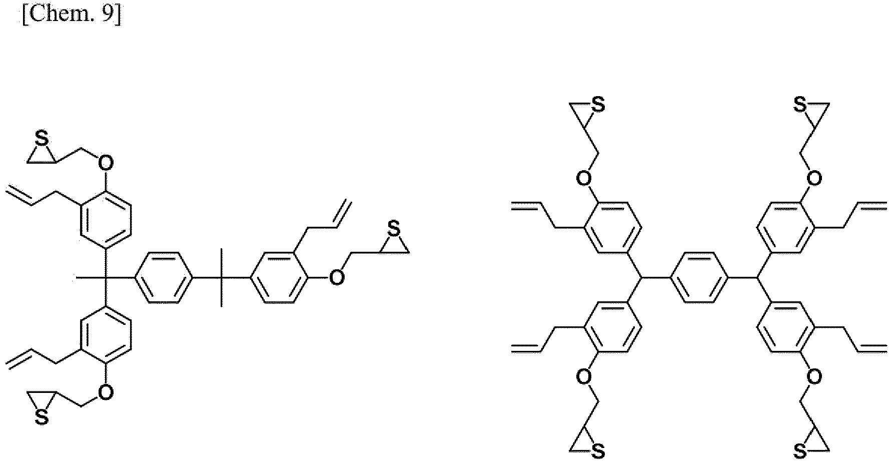

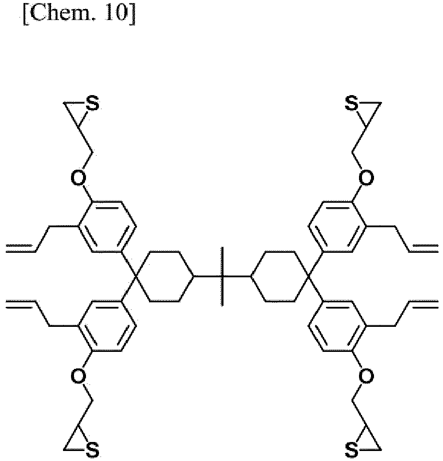

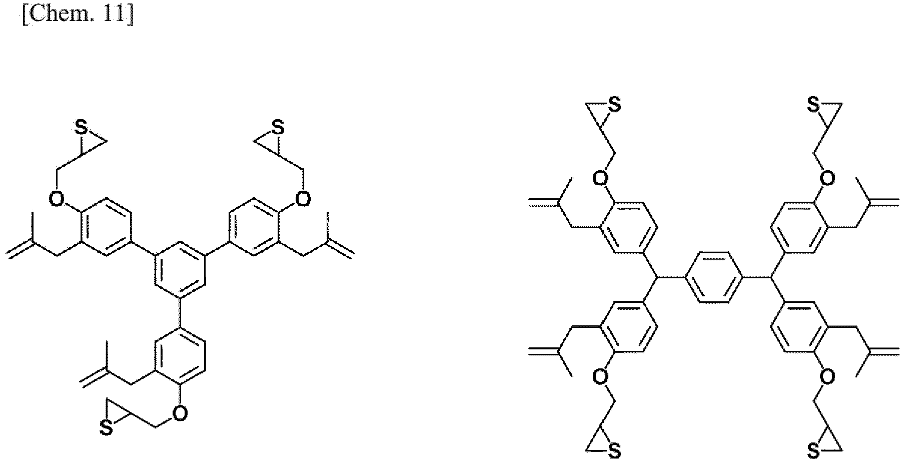

The compound of formula (1) can be synthesized by reacting a glycidyl group and (meth)allyl group-containing compound of formula (2) below with a thiourea or a thiocyanate. 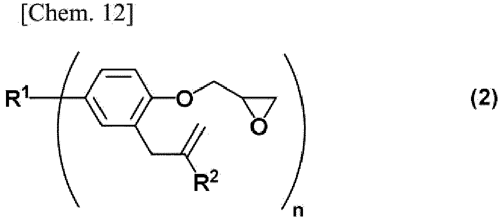 (wherein R¹, R² and n are the same as defined above.)

Examples of the compound of formula (2) include, but are not limited to, those shown below.

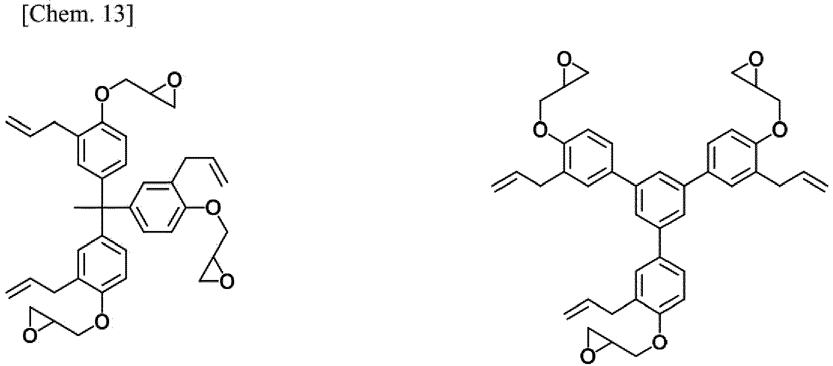

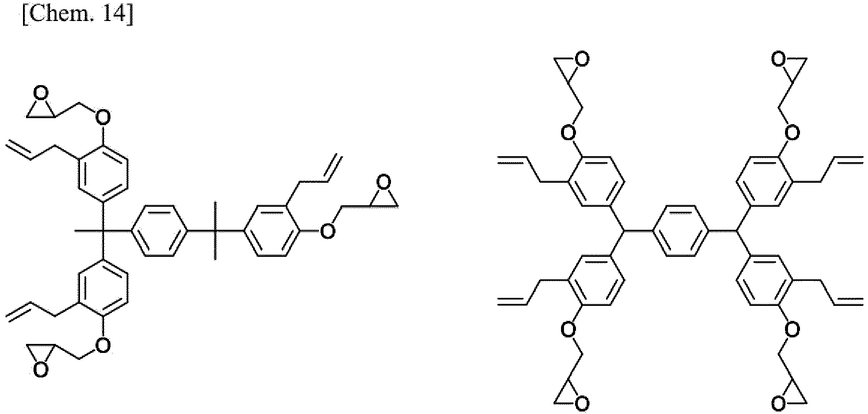

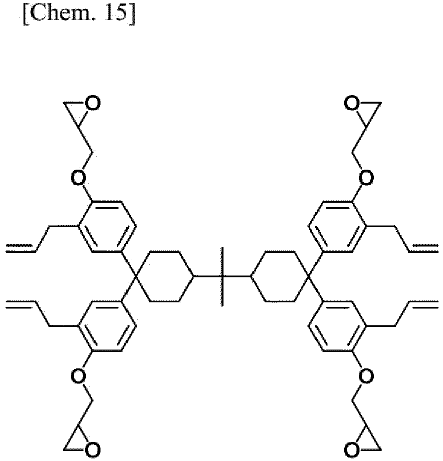

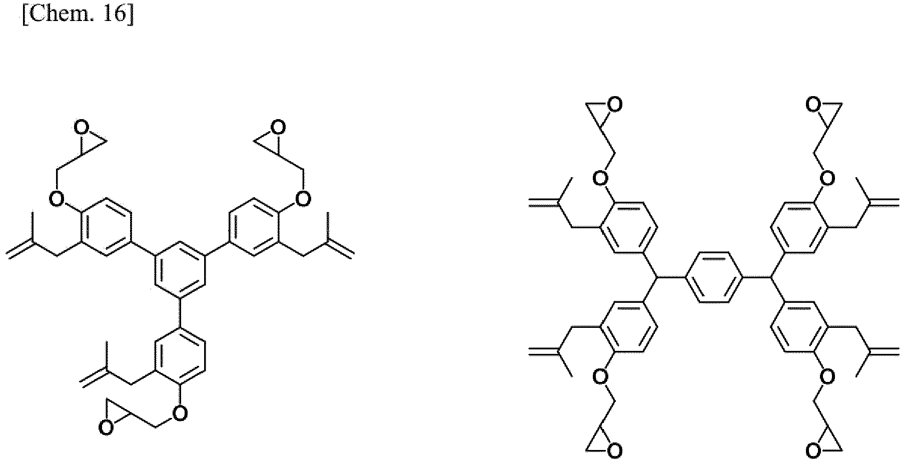

The temperature of the reaction between the compound of formula (2) and a thiocyanate or a thiourea, although not particularly limited, is generally between 0°C and 100°C, and preferably between 20°C and 70°C. The reaction time is not particularly limited so long as it is a time that allows the reaction to reach completion. A reaction time of 20 hours or less is generally appropriate.

The thiocyanate and thiourea are used in a molar amount that stoichiometrically corresponds to the molar amount of epoxy groups on the epoxy compound. However, where product purity, reaction rate, cost effectiveness or the like is of particular importance, the thiocyanate or thiourea may be used in an amount that is lower than this or may be used in an amount that is higher than this. The amount of thiocyanate or thiourea reacted per mole of epoxy groups on the epoxy compound is preferably more than 0 mole and up to 5 moles, more preferably from about 0.9 to about 5.0 moles, and still more preferably from about 1.0 to about 3.0 moles.

The reaction may be carried out in the absence of a solvent or may be carried within a solvent. When a solvent is used, it is preferable to use one in which the thiocyanate or thiourea is soluble or one in which the compound of formula (2) is soluble. Specific examples include water; alcohols such as methanol and ethanol; ethers such as diethyl ether, tetrahydrofuran and dioxane; hydroxyethers such as methyl cellosolve, ethyl cellosolve and butyl cellosolve; aromatic hydrocarbons such as benzene, toluene and xylene; and halogenated hydrocarbons such as dichloroethane, chloroform and chlorobenzene. Mixtures of these solvents are also acceptable. For example, mixed solvents of an alcohol and water, and mixed solvents of an ether, hydroxyether, halogenated hydrocarbon or aromatic hydrocarbon and an alcohol are sometimes effective.

Adding a polymerization inhibitor to the reaction mixture is effective for enhancing the reaction outcome. Exemplary polymerization inhibitors include acids and acid anhydrides. Specific examples of the acids and acid anhydrides include nitric acid, hydrochloric acid, sulfuric acid, fuming sulfuric acid, boric acid, arsenic acid, phosphoric acid, hydrocyanic acid, acetic acid, peracetic acid, thioacetic acid, oxalic acid, tartaric acid, propionic acid, butyric acid, succinic acid, maleic acid, benzoic acid, nitric anhydride, sulfuric acid anhydride, boric oxide, arsenic pentoxide, phosphorus pentoxide, chromic anhydride, acetic anhydride, propionic anhydride, butyric anhydride, succinic anhydride, maleic anhydride, benzoic anhydride, phthalic anhydride, silica gel, silica-alumina and aluminum chloride. Generally, when such a polymerization inhibitor is added, the amount of addition is preferably from 0.001 to 10 wt%, and more preferably from 0.01 to 1 wt%, of the overall reaction mixture. A single polymerization inhibitor may be used by itself, or two or more may be used in combination.

By using an acidic aqueous solution to wash the reaction product, it is possible to increase the stability of the resulting compound. Specific examples of the acid used in the acidic aqueous solution include nitric acid, hydrochloric acid, sulfuric acid, boric acid, arsenic acid, phosphoric acid, hydrocyanic acid, acetic acid, peracetic acid, thioacetic acid, oxalic acid, tartaric acid, succinic acid and maleic acid. These may be used singly or two or more may be used in admixture. The acidic aqueous solution generally exhibits an effect at a pH of 6 or lower, with a pH of 1 to 5 being more effective. The acid may be of one type used alone or two or more may be used in combination.

The thioepoxy group and (meth)allyl group-containing compound of the invention is a compound having at least three thioepoxy groups and at least three (meth)allyl groups per molecule. By utilizing the reactivity of the (meth)allyl groups and carrying out hydrosilylation reactions with a siloxane compound containing Si-H groups to effect polymerization, a thioepoxy group-containing polymeric silicon material having a three-dimensional crosslinked structure can be obtained; by utilizing the reactivity of the thioepoxy groups, use of the product as a curable material having a three-dimensional crosslinked structure is also possible. After polymerization using these groups, crosslinking and curing is possible using the remaining functional groups. Accordingly, the thioepoxy group and (meth)allyl group-containing compound of the invention is a compound useful as a macromonomer for heat-resistant resin materials.

### EXAMPLES

The invention is illustrated more fully below by way of Examples, although the invention is not limited by these Examples.

### [Example 1]

A 3-liter separable flask equipped with a nitrogen gas inlet, a thermometer, a Dimroth condenser, a vacuum controller and an aspirator was charged with 297 g (0.5 mol) of the compound of formula (2-1) below 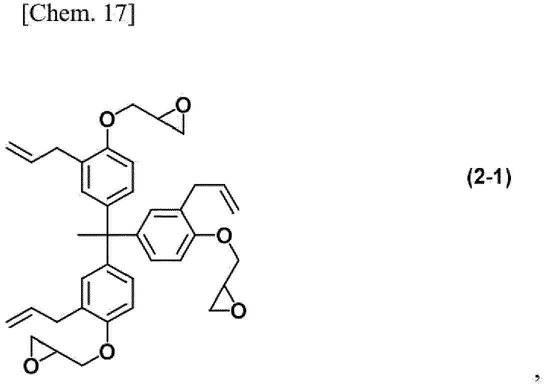 304 g (4 mol) of thiourea, 10 g of acetic anhydride and, as solvents, 1,000 g of toluene and 1,000 g of methanol, and the reaction was carried out at 50°C for 8 hours. Following the reaction, the toluene was extracted and the system was washed with a 1 wt% aqueous solution of sulfuric acid and rinsed with water, after which excess solvent was removed by distillation, giving 305 g of product. Elemental analysis was carried out using the 2400II CHNS/O fully automated elemental analyzer from Perkin Elmer and GC-MS analysis was carried out using the 7890A from Agilent, as a result of which the molecular weight was confirmed to be 642. ¹H-NMR analysis was carried out using the AV400M from Bruker and FT-IR analysis was carried out using the Nicolet 6700 from Thermo Fisher Scientific, based on which the product was confirmed to be the compound of formula (1-1) (yield, 95%).

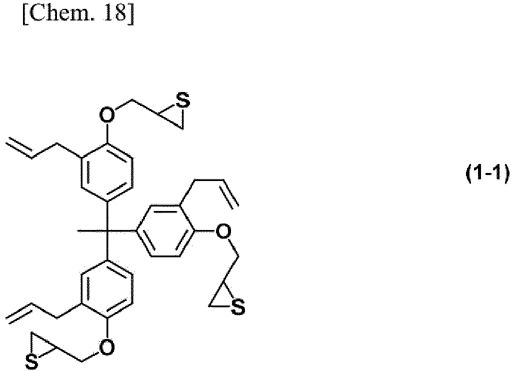

### [Example 2]

Aside from using 321 g (0.5 mol) of the compound of formula (2-2) below instead of the compound of formula (2-1), 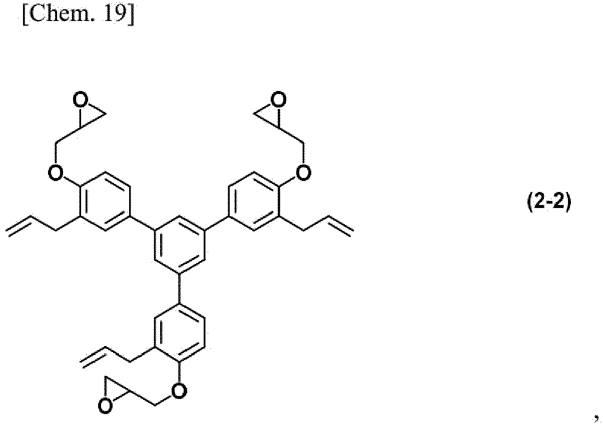 223 g of product was obtained in the same way as in Example 1. Elemental analysis was carried out using the 2400II CHNS/O fully automated elemental analyzer from Perkin Elmer and GC-MS analysis was carried out using the 7890A from Agilent, as a result of which the molecular weight was confirmed to be 690. ¹H-NMR analysis was carried out using the AV400M from Bruker and FT-IR analysis was carried out using the Nicolet 6700 from Thermo Fisher Scientific, based on which the product was confirmed to be the compound of formula (1-2) (yield, 92%).

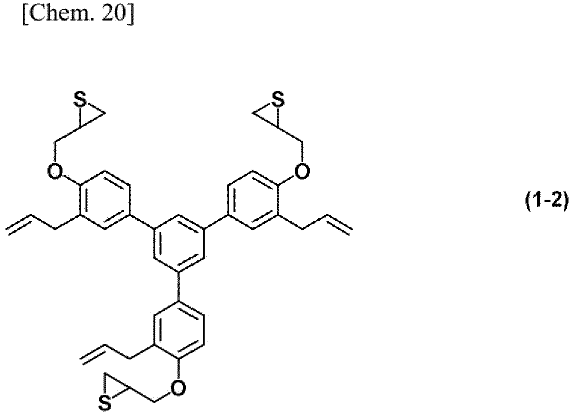

### [Example 3]

Aside from using 457 g (0.5 mol) of the compound of formula (2-3) below instead of the compound of formula (2-1), 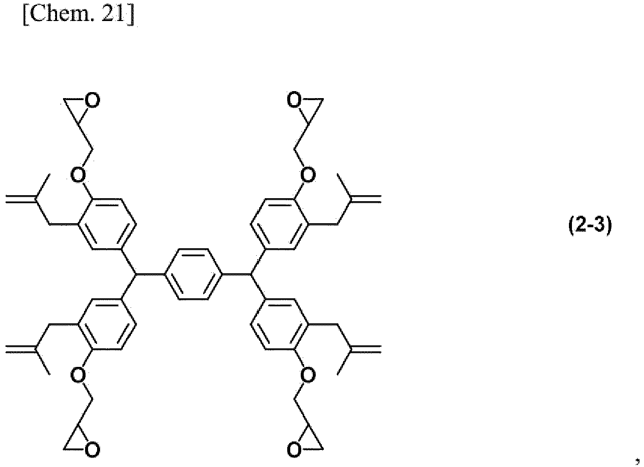 455 g of product was obtained in the same way as in Example 1. Elemental analysis was carried out using the 2400II CHNS/O fully automated elemental analyzer from Perkin Elmer, ¹H-NMR analysis was carried out using the AV400M from Bruker and FT-IR analysis was carried out using the Nicolet 6700 from Thermo Fisher Scientific, as a result of which the product was confirmed to be the compound of formula (1-3) (yield, 93%).

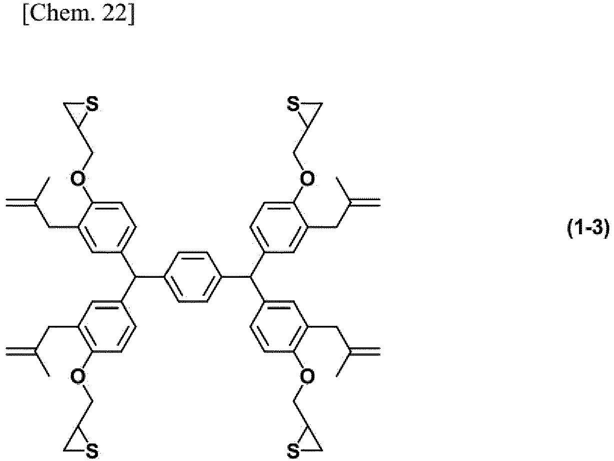

Using the compounds obtained in Examples 1 to 3, the bisphenol A-type epoxy resin jER828, a phenolic curing agent and, as the catalyst, triphenylphosphine (TPP), 2 hours of curing was carried out at 175°C, thereby producing crosslinked resins. The refractive indices of the resulting resins were measured, as a result of which each crosslinked resin was confirmed to exhibit a high refractive index of between 1.65 and 1.70. In addition, each of the resins was confirmed to have a high modulus of elasticity of between 2,000 and 3,000 MPa and a high flexural strength of between 100 and 150 MPa. Also, 250°C heat resistance tests were carried out, as a result of which there were no problems whatsoever with these crosslinked resins, confirming them to have a high heat resistance.

## Claims

1. A thioepoxy group and (meth)allyl group-containing compound of formula (1) below 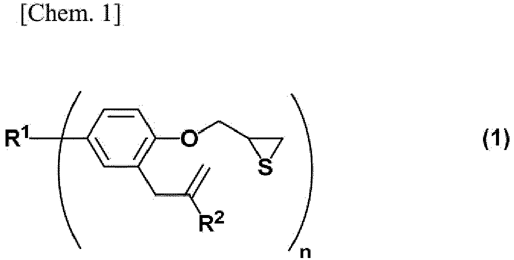 (wherein R¹ is a trivalent or tetravalent hydrocarbon group of 2 to 20 carbon atoms, R² is a hydrogen atom or a methyl group, and n is 3 or 4).

2. The thioepoxy group and (meth)allyl group-containing compound of claim 1, wherein R¹ is a trivalent or tetravalent hydrocarbon group of 2 to 15 carbon atoms.

3. A method for preparing a thioepoxy group and (meth)allyl group-containing compound of formula (1) below 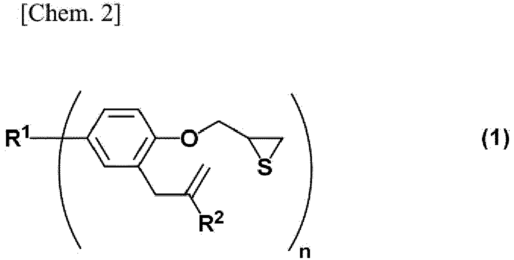 (wherein R¹ is a trivalent or tetravalent hydrocarbon group of 2 to 20 carbon atoms, R² is a hydrogen atom or a methyl group, and n is 3 or 4), which method includes the step of reacting a glycidyl group and (meth)allyl group-containing compound of formula (2) below 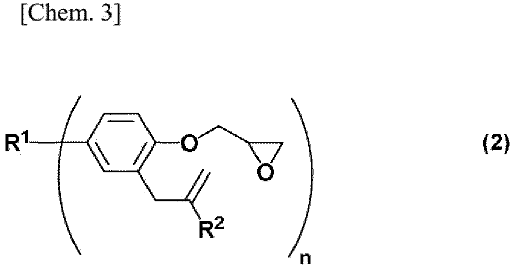 (wherein R¹ is a trivalent or tetravalent hydrocarbon group of 2 to 20 carbon atoms, R² is a hydrogen atom or a methyl group, and n is 3 or 4) with a thiourea or a thiocyanate.
